# EUROPEAN PATENT APPLICATION

(11) **EP 0 711 601 A2**
(43) Date of publication of application: **15.05.1996**
(21) Application number: 95308011.6
(22) Date of filing: 09.11.1995
(51) Int. Cl.: B01J 29/08, B01J 29/04, C07C 41/09

(54) **Catalyst for multistage etherification with high conversion of T-butanol**

(30) Priority: 14.11.1994 US 338079
(71) Applicant: TEXACO DEVELOPMENT CORPORATION, White Plains, New York 10650 (US)
(72) Inventor: Dai, Pei-Shing Eugene, Port Arthur, Texas 77642 (US); Knifton, John Frederick, Austin, Texas 78726 (US)
(74) Representative: Watkins, David

(57) **Abstract**

A catalyst composition is disclosed which exhibits improved hydrothermal stability and stability to higher temperatures. It is useful, for example, in a second stage reactor for improved conversion of C₄ - C₁₀ t-alcohol/C₁ - C₆ primary alcohol to the corresponding alkyl tertiary alkyl ether. The catalyst is a composition selected from (i) a strongly acidic Y-zeolite having a silica:alumina ratio of 300:1 to 1:1 and a unit cell size in the range of 24.20 to 24.67Å and (ii) a silicoaluminophosphate having a pore size from 3Å to 9Å, modified with a fluoride-containing compound.

## Description

The present invention relates to the generation of MTBE/ETBE from crude t-butanol/methanol feedstocks. More particularly, it is concerned with a catalyst composition comprising acidic, fluoride-treated Y-zeolites, having defined physical characteristics, which are employed in a second stage reactor that receives the bottoms from a primary fractionator of a unit for making MTBE, or other oxygenates, and further reacts the bottoms at a temperature greater than 120°C to increase the total t-butanol conversion, up to greater than 90% in a continuous unit.

In addition, the acidic, fluoride-treated Y-zeolite catalysts allow phase separation in the second stage at temperatures in the range of 160°C to 220°C into an isobutylene/MTBE product rich phase and a heavier aqueous methanol phase.

It is known to those skilled in the art that ethers, including unsymmetrical ethers, may be prepared by reacting an alcohol with another alcohol to form the desired product. The reaction mixture, containing catalyst and/or condensing agent, may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently most commercial processes for the manufacture of methyl tert-butyl ether are based upon the liquid-phase reaction of isobutylene and methanol (see Equation 1 below), catalysed by a cationic ion-exchange resin. The cationic ion-exchange resins used in MTBE synthesis normally have sulphonic acid functionality.

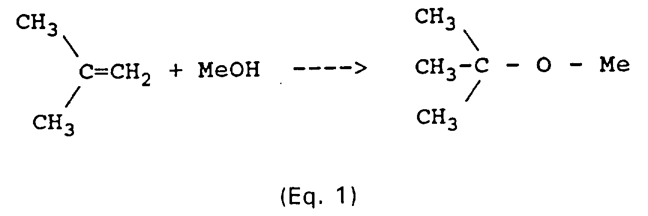

With the expanding use of MTBE as an acceptable gasoline additive, a growing problem is the availability of raw materials. Historically, the critical raw material has been isobutylene. Recently, however, United States Patent Nos. 5,099,072, 5,169,592 and 5,081,318, among others, have disclosed a one-step method for producing MTBE from t-butanol (tBA) over various catalysts. It would be advantageous to obtain additional conversion in the crude feedstock without having to recycle unconverted t-butanol.

The preparation of MTBE from methanol and t-butanol is discussed in a paper by S. V. Rozhkov *et al.,* Prevrashch Uglevodorodov, Kislotno-Osnovn. Geterogennykh Katal. Tezisy Dokl. Vses Konf., 1977, 150 (C. A. 92:58165y). Here, the t-butanol and methanol undergo etherification over KU-2 strongly acidic sulphopolystyrene cation-exchangers under mild conditions. This reference contains data on basic parameters of such a process. It is also noted that, although a plant for etherification over cation exchangers does not present any major problems, important consideration must be given to the fact that recycling large amounts of t-butanol and methanol, as well as isobutylene, causes the scheme to be somewhat expensive. Also, the progress of the reaction over cation exchangers is usually complicated by various adsorption and diffusion factors, by swelling phenomena, and by the variable distribution of the components between the solution and ion-exchanger phase. Moreover, acidic cation-exchangers with an organic (polystyrene or polymethacrylate) backbone generally have a very limited stability range with regard to operating temperatures, with temperatures above 120°C normally leading to irreversible destruction of the resin and loss of catalytic activity.

The article entitled "Preparation of Methyl Tert-Butyl Ether (MTBE) Over Zeolite Catalysts" by Pochen Chu and Günther H. Kühl in Ind. Eng. Chem. Res., 26, 365, (1987) discloses work which identifies ZSM-5 and ZSM-11 to be selective for the preparation of MTBE. Compared to the conventional Amberlyst 15 resin, the zeolites are thermally stable and give noacid effluent. They provide high selectivity to MTBE with little or no diisobutene yield, are less sensitive to the CH₃OH/i-C₄H₈ ratio and exhibit good selectivity even at ratios approaching unity. They provide high MTBE output, despite the unfavorable thermodynamic equilibrium, since the process utilising these zeolites can be operated at high temperature and high space velocity. In addition, deactivation is not observed in short catalytic tests, nor is reactivation required. The excellent selectivity of these two zeolites is believed to be due to the size and structure of their pores, which provide easy access to methanol and restricted access to isobutene. Zeolite beta was also tested, giving the poorest results, and small pore zeolites were inactive. As expected, large pore zeolites did not exhibit shape selectivity.

Another important aspect regarding zeolites involves methods of generating acidity. Several ways of introducing acidity into a zeolite are known in the art and they result in the formation of Bronsted acid sites. The total acidity of a zeolite catalyst depends on both the concentration of acidic sites and the strength of the individual sites. The number and nature of active sites in a zeolite catalyst can be determined in several ways, including ⁷Al solid state NMR, uptake of base and poisoning experiments. Maximum overall acidity is often found for Si/Al ratios in the range of 5 to 20. Bronsted acid sites formed by various methods can form Lewis acid sites by dehydroxylation:

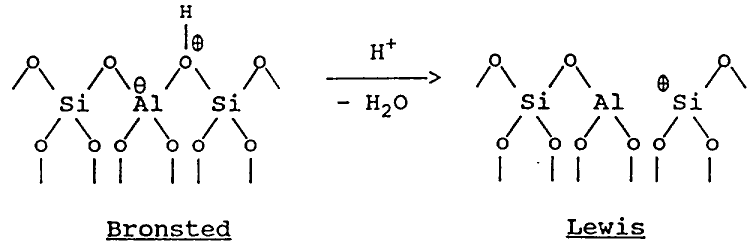

Where shape selectivity by size exclusion is the key to zeolite function, it can be accomplished either through reactant selectivity or product selectivity. It is believed Columbic field effects may also play a part. Another phenomenon which has been observed to contribute is configurational diffusion, which occurs in situations where structural dimensions of the catalyst approach those of molecules. Even subtle changes in dimensions of molecules can result in large changes in diffusivity.

Another type of selectivity which has been observed is spatiospecificity or restricted transition state, where both the reactant molecule and the product molecule are small enough to diffuse through channels, but the reaction intermediates are larger than either the reactants or the products and are spatially constrained.

Reactants which are of particular interest in shape selective catalysis include straight-chain and slightly branched paraffins and olefins, naphthenes and aromatics.

In United States Patent No. 4,943,545, there is suggested modification of Y-zeolites having Si:AI ratio of at least 4 with a very dilute (0.001 → 0.1 N) solution of HF in a cracking catalyst as a means of reactivation.

United States Patent Nos. 5,099,072; 5,169,592 and 5,081,318 relate to various zeolite catalysts used in the one-step synthesis of MTBE from t-butanol.

In United States Patent No. 5,300,697 there is disclosed a catalyst for synthesis of MTBE from t-butanol comprising hydrogen fluoride modified zeolites.

In United States Patent No. 5,220,078, there is disclosed a catalyst for MTBE synthesis comprising fluorophosphoric acid-modified zeolites.

In the art, where the feedstock for producing MTBE is t-butanol, the conversions are not as high as would be desirable. It would be a distinct advance in the art if a catalyst was available which could withstand sustained operating temperatures of greater than 200°C and which could be used in a second stage reactor to improve the percentage of conversion of t-butanol to MTBE without the necessity of recycling.

It would be a substantial advance in the art if such a catalyst made it possible to obtain greater than 90% conversion of t-butanol, crude t-butanol/methanol feedstocks at low energy cost.

In accordance with the present invention, there is provided a catalyst having improved hydrothermal stability and stability to higher operating temperatures useful in the reaction between a C₁ - C₆ primary alcohol and a C₄ - C₁₀ tertiary alcohol for producing corresponding alkyl tertiary alkyl ethers, characterised in that said catalyst comprises a composition selected from:
(a) a strongly acidic Y-zeolite having a silica:alumina molar ratio of 300:1 to 1:1 and a unit cell size of 24.20 to 24.67 Å, and
(b) a silicoaluminophosphate having a pore size from 3 to 9 Å,
modified with a fluoride-containing compound.

Optionally, the catalyst composition may be mixed with a binder selected from Group III or Group IV oxides. The catalyst is particularly effective when used in a second stage reactor.

The preferred silicoaluminophosphate (SAPO) molecular sieves which may be used include, but are not limited to, large pore SAPOs such as SAPO-37 and SAPO-5, medium pore SAPOs such as SAPO-11 and SAPO-31.

Examples demonstrate the particular effectiveness of a catalyst composition consisting essentially of a Y-zeolite modified with a compound selected from a fluoride-containing compound, flurosulphonic acid or its cogeners, or triflic anhydride. The catalyst has improved hydrothermal stability and has proven to be active after 2,000 hours of ongoing study.

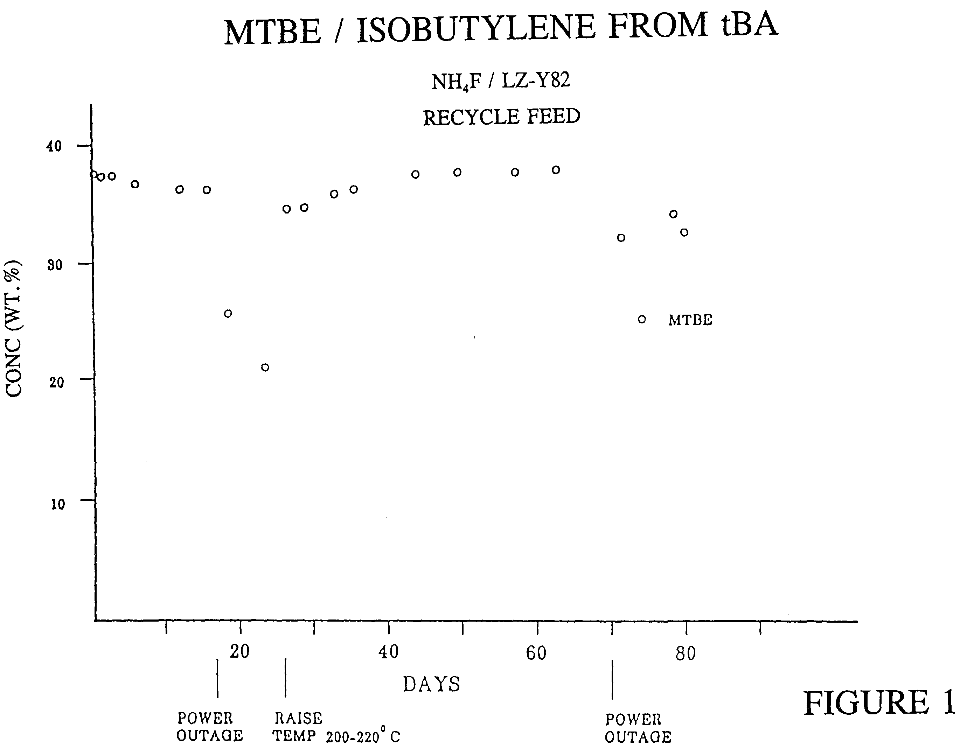

The invention will now be particularly described by way of example only with reference to examples and the single Figure (Figure 1 ) which shows MTBE concentrations in a reactor effluent plotted as a function of time on stream.

The catalysts of the present invention are useful in a process in which there is a primary etherification step using crude t-butanol/methanol to generate MTBE over an acidic catalyst. In order to achieve more complete conversion of t-butanol without recycle, the instant acidic, fluoride-treated Y-zeolite catalyst is used in a second step to etherify remaining t-butanol. The catalysts are particularly effective when used in this way in a second stage reactor.

The effluent from the primary reactor is fed to the MTBE recovery distillation tower which recovers MTBE, isobutylene and some methanol overhead while water, t-butanol and methanol are removed in the bottoms. The bottoms from the primary fractionator, comprising water, t-butanol, methanol and isopropanol, are fed into a second etherification unit and reacted over the acidic fluoride-treated Y-zeolites for additional cogeneration of isobutylene and MTBE. The preferred fluoride-treated Y-zeolites achieve > 90% conversion, are stable at high temperatures, and exhibit greater than 2000 hours of service.

More particularly, the present catalyst composition provides for etherification of the bottoms from the primary fractionator comprising crude t-butanol/methanol feedstock over an inorganic acid catalyst at a temperature in excess of 120°C, e.g. 140°-240°C, to complete the conversion of the remaining t-butanol fraction to MTBE plus isobutylene. The t-butanol/methanol feedstock comprises four principal components, namely water, t-butanol, methanol and isopropanol.

The etherification reaction can be represented by:

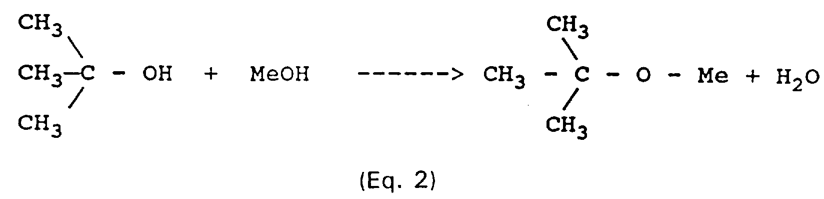

The dehydration reaction can be represented by:

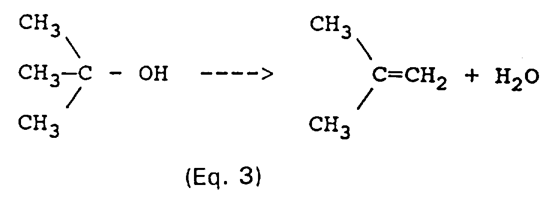

Generally the crude t-butanol/methanol and isopropanol co-reactants may be mixed in any proportion in order to generate the desired MTBE. In the initial etherification, the molar ratio of crude methanol feedstock to t-butanol feedstock in the mixture should be between 10:1 and 1:10 if the yield of MTBE is to be maximised. The most preferred methanol:t-butanol molar ratios are from 1:1 to 5:1. In the present etherification, the proportions of co-reactants are about 2:1. However, in order to achieve maximum selectivity to MTBE, and optimum conversion per pass, an excess of methanol in the liquid feed is desirable.

The syntheses of Equations 2 and 3 can also be conducted when the t-butanol and methanol reactants are mixed with certain other components including water, alcohols such as isopropanol, ketones such as acetone, peroxides and hydroperoxides such as di-t-butyl peroxide and allyl t-butyl peroxide, as well as esters such as t-butyl formate. Typically, each of said classes of components makes up less than 30% of the total feed mixture.

The catalyst can be used in the preparation of other alkyl tertiary alkyl ethers. For example, said catalyst can be used in the reaction of a C₁-C₆ primary alcohol such as methanol, ethanol, n-propanol and n-hexanol with a C₄-C₁₀ tertiary alcohol, such as t-butanol and tertiary amyl alcohol. Reaction of methanol with tertiary amyl alcohol (2-methyl-2-butanol) would then yield methyl tertiary amyl ether (TAME), while reaction of ethanol with t-butanol would yield ethyl t-butyl ether (ETBE). Alternatively, a mixture of alcohols, e.g. a mixture of C₁-C₅ alcohols, could be reacted to give a mixture of alkyl tert-alkyl ethers.

Good results were realised using the catalyst of the present invention which comprise certain acidic, fluoride-treated Y-zeolites as catalysts for the reactions represented in Equations 2 and 3, particularly the isostructural group of faujasite zeolites that include the synthetic Y-zeolites. The preferred Y-zeolites are the ammonium exchanged and rare earth exchanged Y-zeolites.

The unit cells of zeolites X and Y are cubic, a₀ ≈ 2.5 nm, and each contains 192 silicon- or aluminium-centred oxygen tetrahedra which are linked through shared oxygen atoms. Because of the net negative charge on each of the aluminium-centred tetrahedra, each unit cell contains an equivalent number of charge-balancing cations. These are exclusively sodium ions in zeolites in their synthesised form. Typical cell contents for the Y-zeolites in the hydrated form are:

Na₅₆[(A1O₂)₅₆(SiO₂)₁₃₆]250 H₂O

Y-zeolites are distinguished on the basis of the relative concentration of silicon and aluminium atoms and the consequent effects on detailed structure and related chemical and physical properties. The aluminium atoms in the unit cell of Y-zeolite vary from 76 to 48 giving a Si:AI ratio between 1.5 and 3.0. Both the cation concentration and charge density on the aluminosilicate structure are lower for Y-zeolites than for X-zeolites, where the aluminium atoms in the unit cell vary from 96 to 77.

The feature which determines the difference between faujasites and other zeolites built up from sodalite units is the double 6-membered ring or hexagonal prism, by which the units are linked. The sodalite unit, or β-cage, can be represented by a truncated octahedron, with the 24 silicon or aluminium atoms (designated T atoms) taking positions at the vertices. The 36 oxygen atoms are displaced from the midpoints of the edges joining the vertices in order to attain tetrahedral configuration around the T atoms. The free diameter of the void within the β-cage is 0.66 nm, but only the smallest molecules can enter through the 0.22 nm diameter opening in the distorted ring of six oxygen atoms associated with each hexagonal face. Each sodalite unit is linked tetrahedrally across hexagonal faces by six bridging oxygens to four other sodalite units. The larger void spaces enclosed by sodalite units and hexagonal prisms are termed α-cages, or supercages. The α-cage is a 26-hedron with a free diameter of ≈ 1.3 nm, and it can be entered through four distorted 12-membered rings of diameter 0.80-0.90 nm. In this way, each α-cage is tetrahedrally joined to four others giving a complex system of void space extending throughout the zeolite structure. The α- and β-cages together give Y-zeolites, along with X-zeolites, the largest void volume of any known zeolites. This is about 50 % by volume of the dehydrated crystal. From the catalytic viewpoint, the α-cages are by far the most important since, unlike the β-cages, they permit entry of numerous aliphatic and aromatic compounds.

Particularly effective in the subject synthesis of MTBE/ETBE are the synthetic Y-zeolites. Preferably, said zeolites should be in a strongly acidic form whereby some, or all, of the cations (Group I or II, alkali or alkaline earth metal ions such as sodium, potassium, calcium or magnesium) are exchanged by protons, either through ammonium exchange followed by thermal stabilisation (deammoniation, removal of NH₃) at elevated temperatures (e.g. 400-500°C), or through mineral acid treatment, etc. Alternatively, said Y-zeolites may be rare-earth exchanged with, for example, a mixture of rare-earth salts, by treatment with lanthanum salts, etc. Said rare-earth exchanged Y-zeolites would then have a Si:Al ratio of 1.5 to 3. The preferred acidic Y-zeolites for the practice of this invention have silica:alumina ratios in the range 300:1 to 1:1. A suitable range is 100:1 to 5:1. The most preferred range is 50:1 to 7:1.

The unit cell size for the present catalyst may be 24.20 to 24.67 Å. A suitable range is 24.22 to 24.67 Å. The most preferred range is 24.24 to 24.56 Å.

Examples of suitable Y-zeolites for the practice of this invention include: Linde SK-500, a rare-earth exchanged Y-zeolite having a Si:Al ratio of 1.5→2.5; PQ Corporation's CP 304-37, a thermally-stabilised ammonium-exchanged Y-zeolite having a silica:alumina ratio of around 11:1 with a silica-alumina binder; CP 316-26, an ammonium-exchanged Y-zeolite having a silica:alumina ratio of 46:1; LZY-82 from UOP having a silica:alumina ratio of 7.8 and a unit cell size of 24.53Å, and LZY-85 having a silica:alumina ratio of 9.1 and a unit cell size of 24.49Å.

As mentioned above, silicoaluminophosphate (SAPO) molecular sieves are also useful in the present invention. The SAPOs have the general formula:

nR (Siₓ Aly P_{z})0₂ bH₂0

where R is an organic compound and x + y + z = 1.

It is believed that the silicoaluminophosphates which would be most useful in the present invention are SAPO-5, SAPO-37, SAPO-1 1 and SAPO-31. They are all suitable for the co-generation of MTBE/ETBE. Moreover, since they are classified as mildly acidic, it is expected that SAPO materials will provide good selectivity to ethers and generate less undesirable diisobutene at elevated temperatures.

It has been discovered that acidic fluoride-treated Y-zeolites, and SAPOs, have a number of improved properties for the production of MTBE. Most importantly, they possess the stability at high temperatures necessary for a second stage process of the type described herein. The acid useful for modifying the zeolites is selected from the group consisting of fluoride-containing compounds such as ammonium fluoride or silicon hexafluoride compounds, hydrogen fluoride, hydrofluoric acid, or fluorosulphonic acid and its congeners such as fluorosulphonic acid, trifluoromethane sulphonic acid (triflic acid), as well as triflic anhydride. These fluorosulphonic acids can be substituted with a variety of alkyl groups, as in the case of trifluoromethanesulphonic acid. Methods of preparing these triflic acid-modified catalysts are illustrated in Examples 2 and 3 below.

The fluoride-modified zeolites are prepared by treating the Y-zeolite or dealuminised Y-zeolite with hydrogen fluoride, with an aqueous solution of hydrofluoric acid, with ammonium fluoride, or with a solution of HF or NH₄F in a suitable organic solvent. Preferably, the hydrogen fluoride is added to said zeolite as a solution of hydrofluoric acid in distilled water. The method of preparing these HF-modified catalysts is illustrated in Examples 1 and 5.

For example, the treatment of the Y-zeolites with fluoride ion is accomplished by adding a solution of from 1 % to about 60% or 0.1 N to 10 N fluoride compound, such as hydrogen fluoride, ammonium fluoride, triflic acid, fluorosulphonic acid or triflic anhydride in distilled water or an organic solvent such as a ketone, e.g. acetone, to a Y-zeolite or dealuminised Y-zeolite, stirring under nitrogen for at least 1 hour to 24 hours, filtering and washing the solids with distilled water, then drying under vacuum at 40°C overnight and for a further 4 hours at 150°C.

Said catalysts should have a residual acidity in the range 0.1 to 100 mg KOH/g and they may be in the form of powders, pellets, granules, spheres, shapes and extrudates. The examples described herein demonstrate the advantages of using extrudates.

The reaction may be carried out in either a stirred slurry reactor or in a fixed bed continuous flow reactor. The catalyst concentration should be sufficient to provide the desired catalytic effect.

Etherification (Equation 2) and dehydration (Equation 3) using said catalyst can generally be conducted at temperatures from 20 to 300°C; the preferred range is 120 to 250°C and the most preferred range is 180 to 220°C. The total operating pressure may be from 0 to 1000 psig (0.1 to 7 MPa), or higher. The preferred pressure range is 50 to 500 psig (0.4 to 3.5 MPa).

In certain circumstances, it may be particularly desirable that the t-butanol conversion is sufficiently high (e.g. 80% or greater), that the crude product mix phase separates into an isobutylene-MTBE product-rich phase and a heavier aqueous methanol phase. Preferably, such product phase separation is achieved at as low an etherification temperature as possible, but particularly in the range 160-240°C.

Typically, MTBE is generated continuously in proportions up to about 30 % by weight, or greater, in the crude liquid product at total liquid hourly space velocities (LHSV) of 6 or higher and relatively mild conditions, where:$\text{LHSV=} \frac{\text{Volume Of Total Liquid Feed Run Through The Reactor Per Hour}}{\text{Volume of Catalyst In Reactor}}$

Conversions of t-butanol (tBA, wt%) are estimated in the following examples using the equation:$\frac{\left(\text{Wt% Conc. of tBA in Feed-Wt% Conc. of tBA in Product}\right)}{\text{Wt% Conc. of tBA in Feed}} \text{×100}$

Selectivities for MTBE (mole %) and isobutylene (C₄H₈, mole%) are estimated from:$\frac{\text{Moles of MTBE} \left({\text{or C}}_{\text{4}} {\text{H}}_{\text{8}}\right) \text{in Product}}{\text{moles of tBA converted}} \text{×100}$

The examples which follow illustrate the second stage synthesis of MTBE and isobutylene from crude t-butanol/methanol feedstocks using the acidic, fluoride-treated Y-type zeolites, particularly in the form of extrudates. The examples are only intended as a means of illustration and it will be understood by persons skilled in the art that the invention is not limited thereby.

### EXAMPLE 1 (6798-6R)

This example illustrates the preparation of a hydrogen fluoride-modified Y-zeolite.

To 100g of Y-zeolite (CP316-26, an ammonium-exchanged, thermally-stabilised Y-zeolite having a silica:alumina ratio of 46 and a unit cell size of 24:26Å, in 1/16" (1.6 mm) diameter extruded form) was added a (3.8N) solution of 48% hydrofluoric acid (50g) in distilled water (100g). The mixture was stirred overnight under nitrogen and then filtered. The solids were then washed with distilled water, dried in vacuo at 40°C overnight, and for a further 4 hours at 150°C.

The recovered white extrudates were found to comprise on analysis:
Fluoride, 1.6%
Water, 1.2%
Acidity, 20.2 mg KOH/g

### EXAMPLE 2 (6798-5)

This example illustrates the preparation of a triflic acid-modified Y-zeolite.

To 100g of Y-zeolite (CP316-26) which had been dried in vacuo at 175°C for 3 hours and had a water content of 0.54% was added a solution (0.67N) of trifluoromethane sulphonic acid (40g) in dried acetone (400cc, dried over 4Å sieve). The mixture was stirred overnight under nitrogen, filtered and the solids washed with dried acetone then dried in vacuo at 40°C overnight, followed by a further 4 hours at 150°C.

The recovered reddish-brown extrudates were found to comprise by analysis:
Fluoride, 1.6%
Water, 0.54%
Acidity, 29.7 mg KOH/g

### EXAMPLE 3 (6798-3R)

This example illustrates the preparation of a triflic acid-modified Y-zeolite.

To 100g of rare-earth exchanged Y-zeolite (Linde SK-500, having a silica:alumina ratio of 2.9, in 1/16" (1.6 mm) diameter extruded form that had been dried in vacuo at 175°C for 3 hours and had a water content of 0.58%) was added a solution of(0.67N) trifluoromethanesulphonic acid (40g) in dried acetone (400cc, dried over 4A sieve). The mixture was stirred overnight under nitrogen, filtered and the solids washed with dried acetone then dried in vacuo at 40°C overnight, followed by a further 4 hours at 150°C.

The recovered brown extrudates were found to comprise by analysis:
Water, 0.89%
Acidity, 52.6 mg KOH/g

### EXAMPLE 4

Example 4 with Code No. 2143CT90 was prepared by ion exchange of the Y-82 zeolite/alumina extrudates from UOP with 2.5 wt% aqueous ammonium fluoride solution at 60°C for 4 hours and calcination at 593°C for 3 hours.

### EXAMPLE 5

Example 5 with Code No. 6798-6 illustrates the preparation of a hydrofluoric acid-treated Y-zeolite (6.4N).

To 500cc of sample of CP316-26 zeolite/alumina extrudates from PQ Corp. was added 10 vol% HF in distilled water in sufficient quantity to cover the extrudates. The mix was allowed to stand for 1 hour. Excess water was removed by rotary evaporation and the residual solids were dried at 200°C overnight.

### EXAMPLE 6

This example illustrates the co-synthesis of isobutylene and MTBE from a crude aqueous t-butanol/methanol feedstock using the ammonium fluoride-modified Y-82 zeolite obtained in Example 4.

The etherification reaction was conducted in a tubular reactor (1/2" (12.7 mm) id, 12" (305 mm) long) constructed of 316 stainless steel, operated upflow and mounted in a furnace controllable to ± 1.0°C and fitted with pumps allowing flow control to < ± 1 cc/hr. The reactor was also fitted with a pressure regulating device and equipment for monitoring temperature, pressure and flow rate.

The reactor was charged at the beginning of the experiment with 50cc of ammonium fluoride-treated Y-zeolite extrudates (Example 4). A screen of glass wool was placed at the top and bottom of the reactor to ensure that the catalyst remained in the middle portion.

The catalyst bed was treated with a crude t-butanol/methanol feed mix also containing sizeable proportions of water and isopropanol components, upflow, at a feed rate of 50 cc/hour, while the reactor was held at 140°C with a total pressure of 300 psi 2.1 MPa). Samples of effluent were collected periodically on stream and analysed by glc and gc-ir.

Typical analyses for samples taken under these conditions are summarised in Table V. Performance at higher temperatures (160°, 180°, 200°C) was determined using the same procedures. These results are also given in Table V.

Operating at 200°C, where the effluent comprised two phases, the t-butanol conversion level and isobutylene/MTBE selectivities were as follows:
t-Butanol conversion = 93%
Isobutylene selectivity = 90 mole%
MTBE selectivity = 7 mole%

The results demonstrate that a two-stage etherification process employing the catalysts of this invention, such as are demonstrated in Examples 4 and 5, in the second-stage reactor facilitates high conversion of t-butanol at temperatures of 120°-200°C. The catalyst also demonstrates a good lifetime activity and hydrothermal stability at high temperature.

A summary of the performances of the five best candidates (giving >90% T-butanol conversion at 200°C) and the performance of ten catalyst samples at 160°C are given in Tables I and II below.

**TABLE I**

| **Catalytic Activities of Zeolite Samples for MTBE Synthesis at 200 °C** | | | | |
|---|---|---|---|---|
| **Sample Code No. (200 °C)** | **Catalyst Description** | **tBA Conv. (%)** | **Molar Select. (%)** | |
| | | | **C₄H₈** | **MTBE** |
| 2143-CT-90 | NH₄F/Y-82 | 93 | 90 | 10 |
| 2141-CT-90 | NH₄F/Y-82 | 93 | 91 | 9 |
| 6798-6 | HF/CP316-26 | 94 | 90 | 7 |
| 6798-3 | TF/SK-500 | 91 | 87 | 13 |
| 6798-5 | TF/CP316-26 | 94 | 94 | 6 |

**TABLE II**

| **Catalytic Activities of Zeolite Samples for MTBE Synthesis at 160 °C** | | | | |
|---|---|---|---|---|
| **Sample Code No. (160 °C)** | **Catalyst Description** | **tBA Conv. (%)** | **Molar Select. (%)** | |
| | | | **C₄H₈** | **MTBE** |
| 2143-CT-90 | NH₄F/Y-82 | 64.6 | 34 | 66 |
| 2142-CT-90 | NH₄F/Y-82 | 45.8 | 41 | 59 |
| 2141-CT-90 | NH₄F/Y-82 | 66.9 | 35 | 65 |
| 6798-6 | HF/CP316-26 | 63.0 | 36 | 64 |
| 6798-3 | TF/SK-500 | 59.8 | 36 | 64 |
| 6798-4-1R | HF/SK-500 | 53.8 | 36 | 64 |
| 6798-5 | TF/CP316-26 | 63.7 | 40 | 60 |
| 6798-7 | HF/CP304-37 | 59.4 | 37 | 63 |
| 2140-CT-90 | NH₄F/Y-85 | 60.0 | 36 | 64 |
| 6798-41 | TA/SK-500 | 60.0 | 37 | 63 |

**TABLE III**

| **Performance of Ammonium Fluoride-Treated Catalyst (Example 1,2143-CT-90)** | | | | | |
|---|---|---|---|---|---|
| **Sample Code No.** | **Time On Stream (days)** | **Operating Temp. (°C)** | **tBA Conv. (%)** | **Molar Select. (%)** | |
| | | | | **C₄H₈** | **MTBE** |
| 6945-23-2R | 2 | 200 | 93 | 89 | 8.7 |
| 6945-23-6 | 17 | 200 | 89 | 81 | 14 |
| 6945-23-14 | 52 | 220 | 92 | >99 | 12 |
| 6945-23-18 | 81 | 220 | 82 | 96 | 24 |

**TABLE IV**

| **Performance of Hydrofluoric Acid-Treated CP316-26 Catalyst (Example 2,6798-6R)** | | | | | |
|---|---|---|---|---|---|
| **Sample Code No.** | **Time On Stream (days)** | **Operating Temp. (°C)** | **tBA Conv. (%)** | **Molar Select. (%)** | |
| | | | | **C₄H₈** | **MTBE** |
| 6945-67-1 | 1 | 200 | 93 | 59 | 7.2 |
| 6945-67-4 | 19 | 220 | 82 | 95 | 18 |
| 6945-67-9 | 53 | 220 | 80 | 89 | 23 |
| 6945-67-14 | 85 | 220 | 80 | 84 | 16 |

### Catalyst Screening Studies

A total of ten fluoride-treated zeolites were evaluated in the subject process. These included Y-zeolites such as:
LZY-82, LZY-85, CP 316-26, CP 304-37 and SK-500 treated with:
ammonium fluoride, HF, triflic acid and triflic anhydride.

The feedstock used in this evaluation had the approximate composition of 25.8% water, 36.9% methanol, 10.6% 2-propanol and 26.0% t-butanol. This simulated the composition of the bottoms from the primary fractionator, downstream of the first stage etherification reactor.

In the first study, a sample of ammonium fluoride-treated Y-82 zeolite, gave t-butanol conversions at 180°C and 200°C, as measured by GLC, of 88% (Sample 6834-64-6) and 93% (Sample 6834-64-7, see Table V), respectively. At both temperatures, the effluent product appeared as two phases - a lighter isobutylene/MTBE product phase and a heavier aqueous alkanol phase. In this experimental series, a sight glass was used to measure the relative sizes of these phases so that isobutylene and MTBE molar selectivities could be accurately calculated. Gas chromatograph-mass spec. analyses of Sample 6834-64-3 indicated the formation of small quantities of diisobutene and isopropyl t-butyl ether (IPTBE).

Two other ammonium fluoride-treated Y-zeolites were also screened in a similar manner (see Tables VI and VII). Sample 2042-CT-90 appeared to deactivate at the higher screening temperatures (180°-200°C), as shown by the figures in Table VI). T-butanol conversions were < 30% and there was no product phase separation. A repeat run gave similar results. Sample 2141-CT-90 performed like the first sample of NH₄F modified Y-82 (cf. Tables V and VII). Two phase effluent products were observed at both 180° and 200°C and for sample 6834-75-R4 the calculated t-butanol conversion was 93% and the isobutylene/MTBE molar selectivities 95 and 9.1%, respectively.

Five fluoride-treated Y-zeolites were then tested in the same service. These solid acids included:
a) HF-treated CP316-26, 1/16" (1.6 mm) extrudates, (Sample 6798-6, Run 6834-81, see Table VIII).
b) TF-treated SK-500, 1/16" (1.6 mm) extrudates, (Sample 6798-3, Run 6834-84, see Table IX).
c) HF-treated SK-500, 1/16" (1.6 mm) extrudates, (Sample 6798-4, Run 6834-93, see Table X).
d) TF-treated CP316-26, 1/16" (1.6 mm) extrudates, (Sample 6798-5, Run 6834-96, see Table XI).
e) HF-treated CP304, 1/16" (1.6 mm) extrudates, (Sample 6798-7, Run 6945-11, see Table XII).

The HF/CP316-26 gave excellent performance - 94% t-butanol conversion per pass at 200°C (Sample 6834-81-R4), with 91% isobutylene selectivity and 7.2% MTBE. Similarly, the triflic acid (TF)-modified SK-500 gave 91 % t-butanol conversion at 200°C (Sample 6834-84-R4). The DME and diisobutene concentrations in these samples are typically <400 ppm for Sample 6834-84-R4 and the isopropyl t-butyl ether (IPTBE) content is 0.2%. The triflic acid-treated dealuminised Y from PQ (Sample 6798-5) also gave 94% t-butanol conversion at 200°C operating temperatures.

Other solid acids evaluated for this purpose include another ammonium fluoride-modified Y-85 and a triflic anhydride (TA) treated SK-500. The results of these runs are given in Tables XIII and XIV.

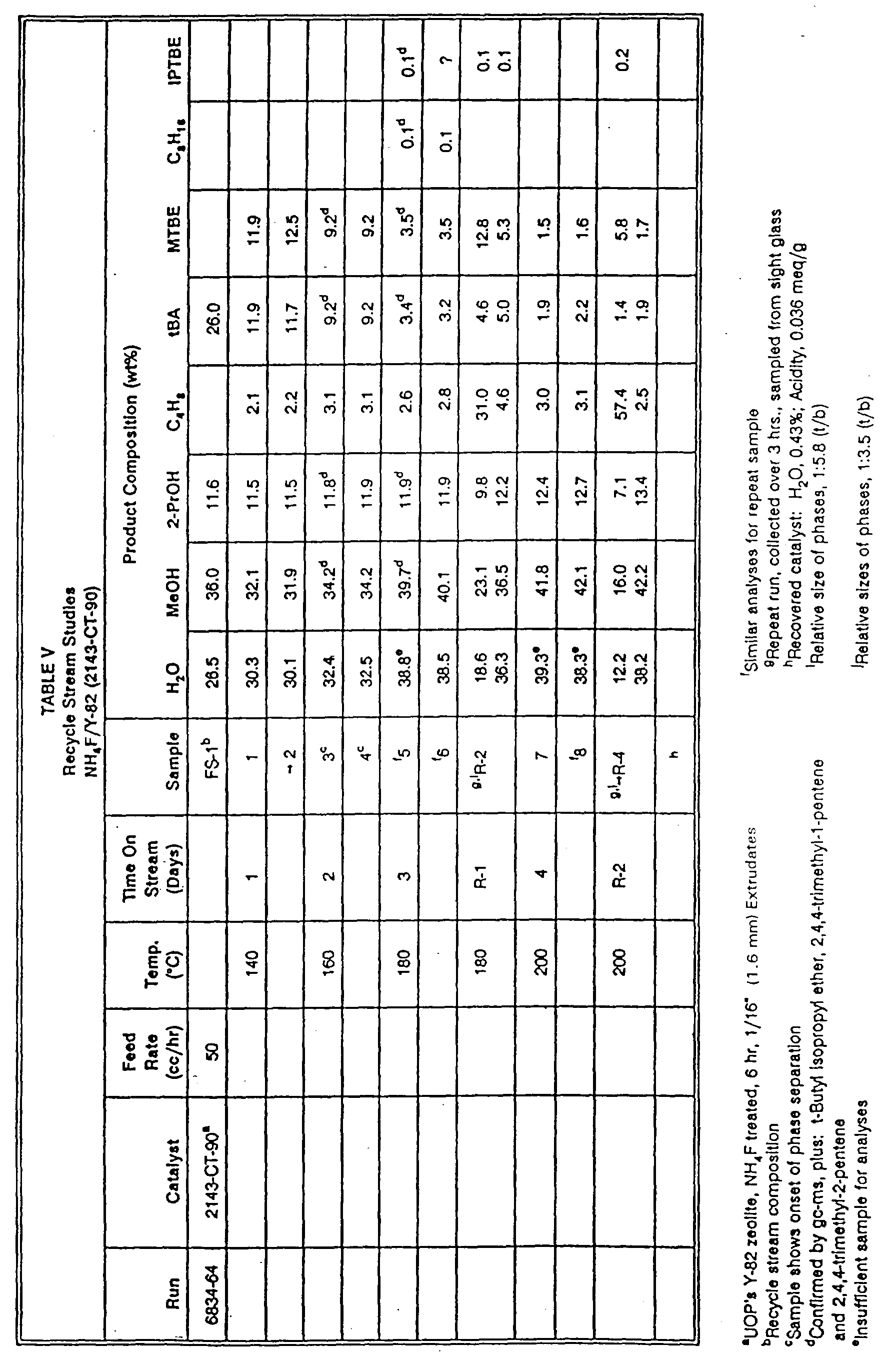

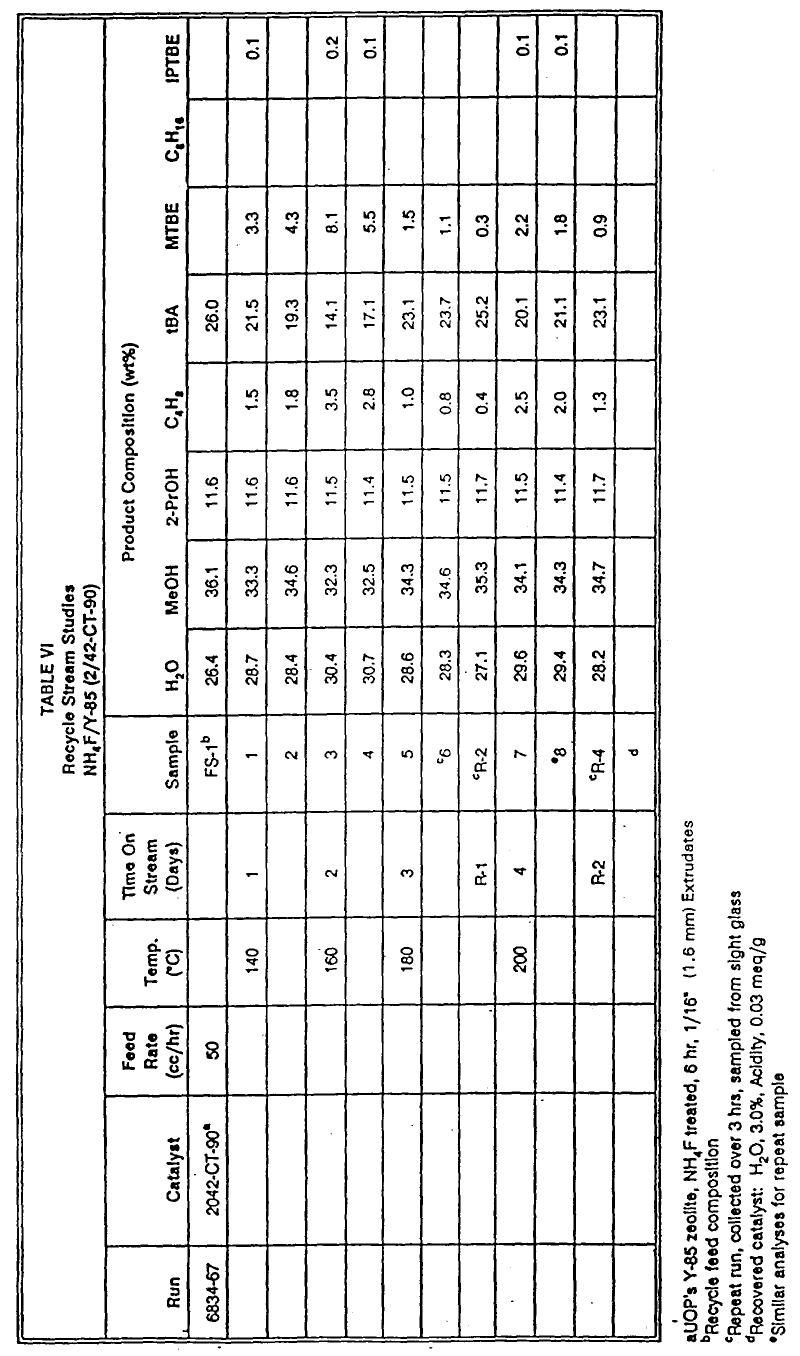

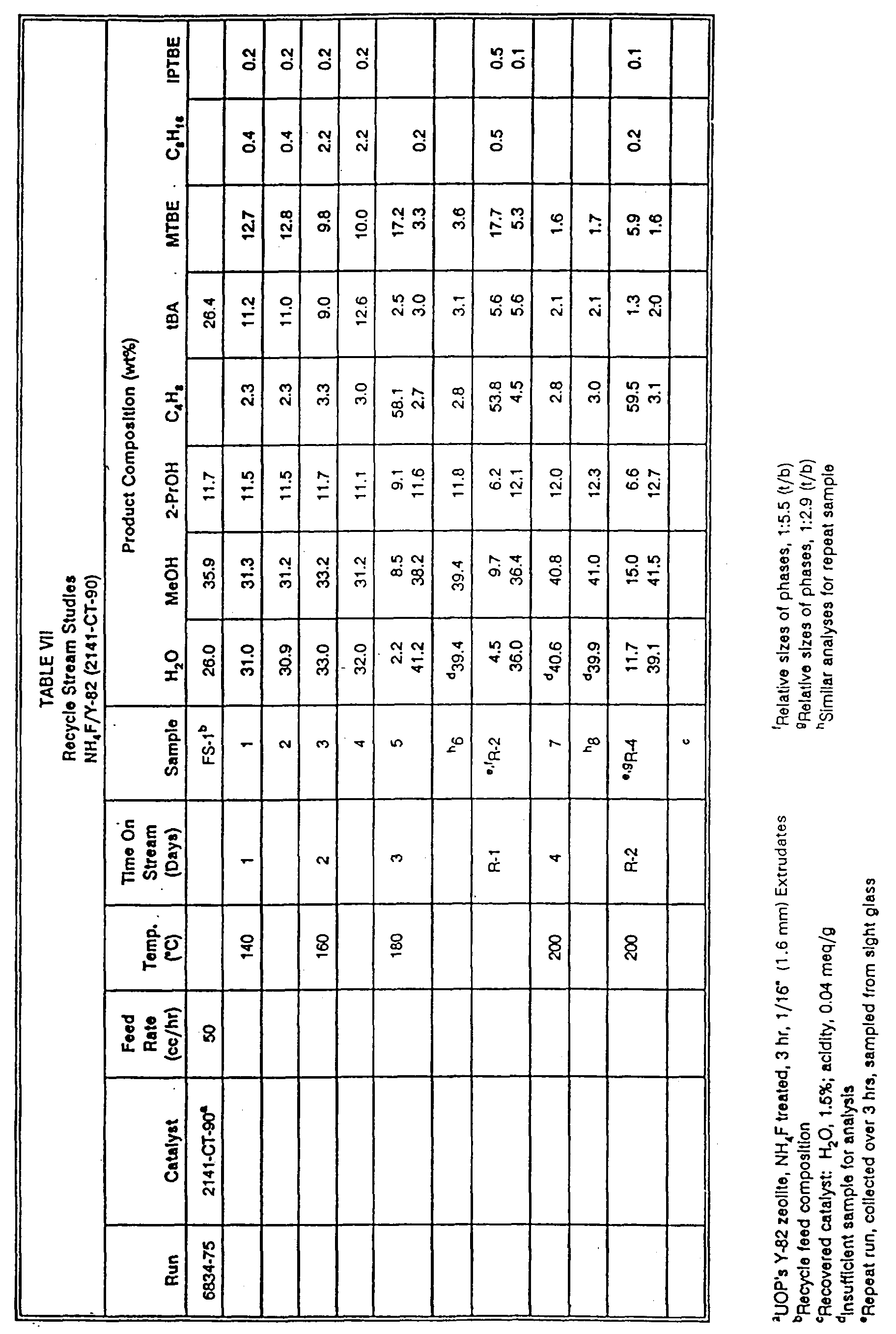

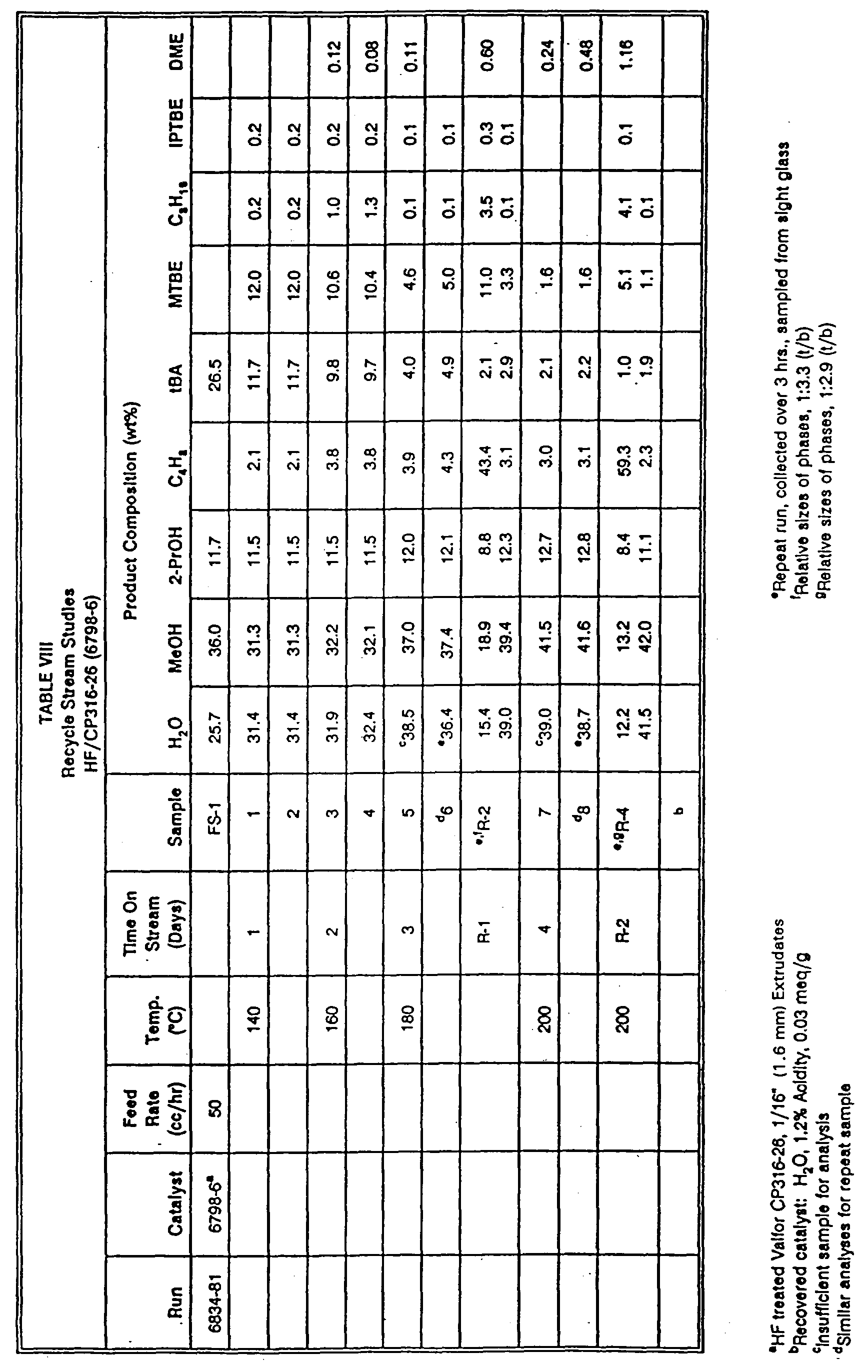

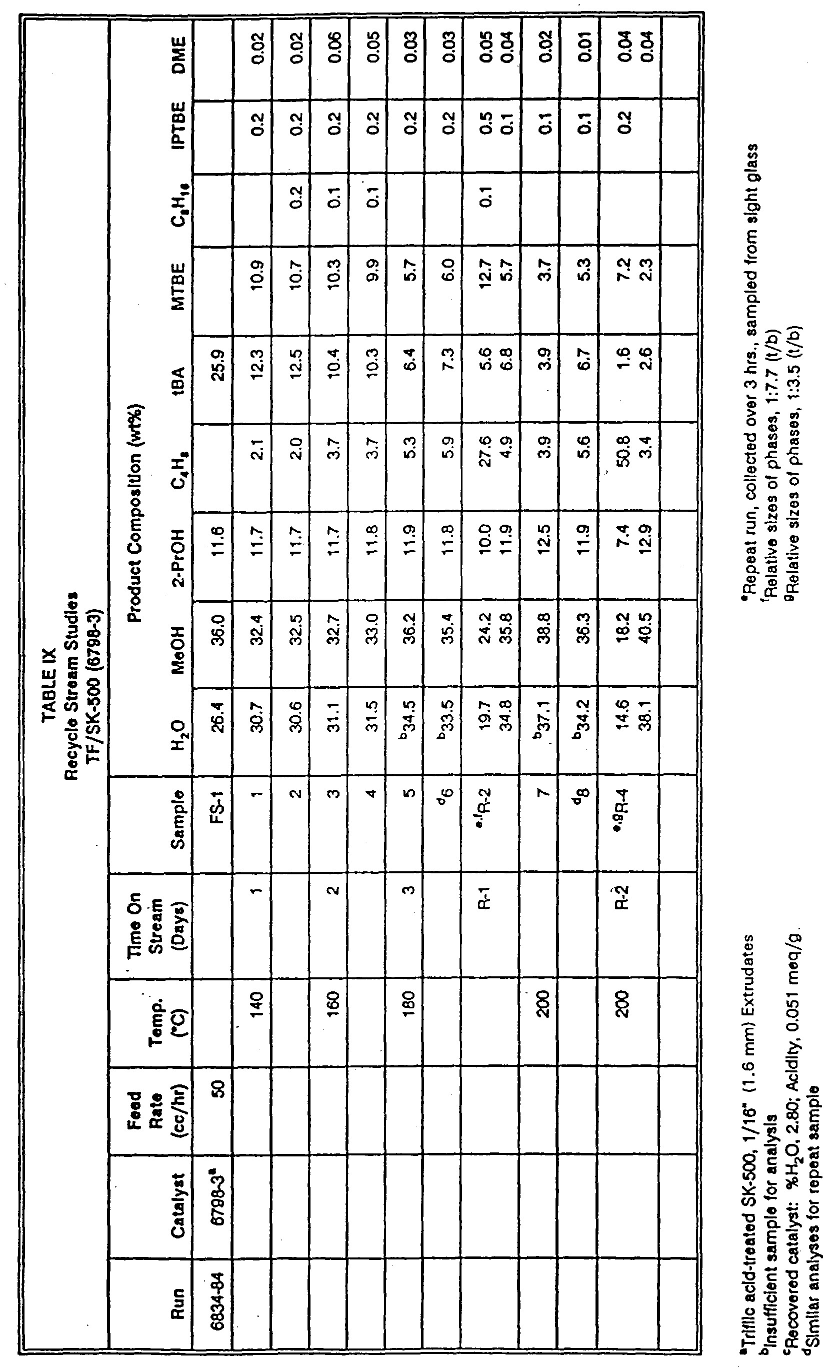

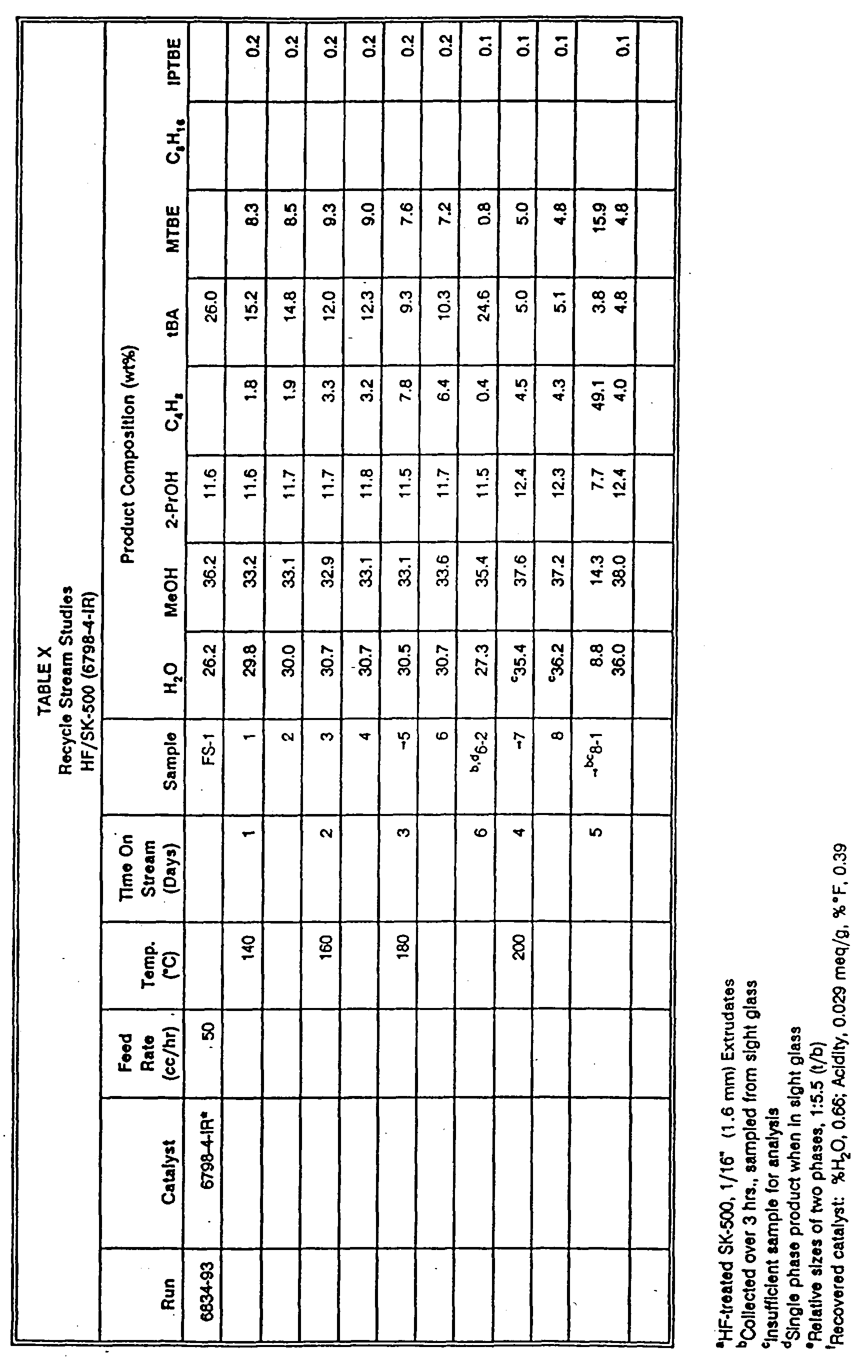

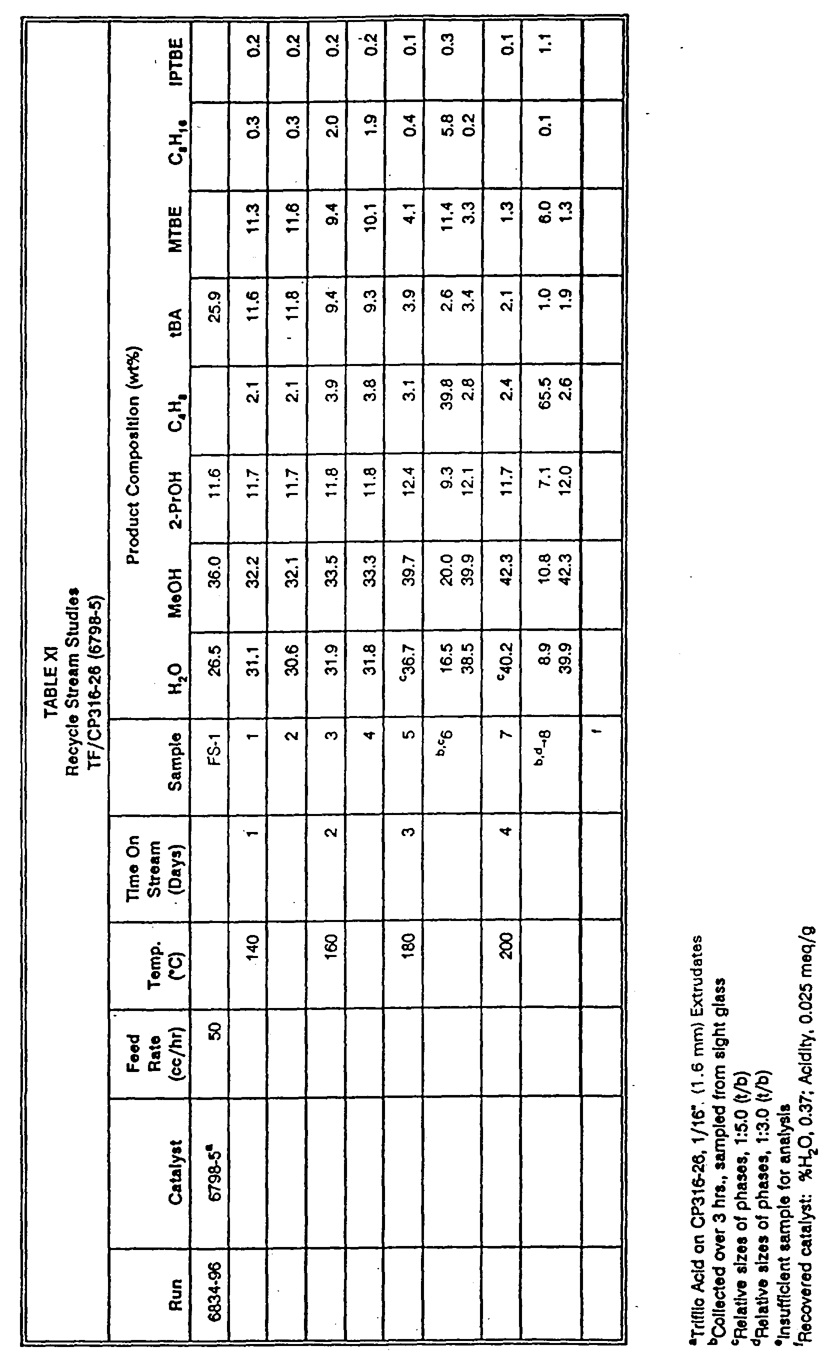

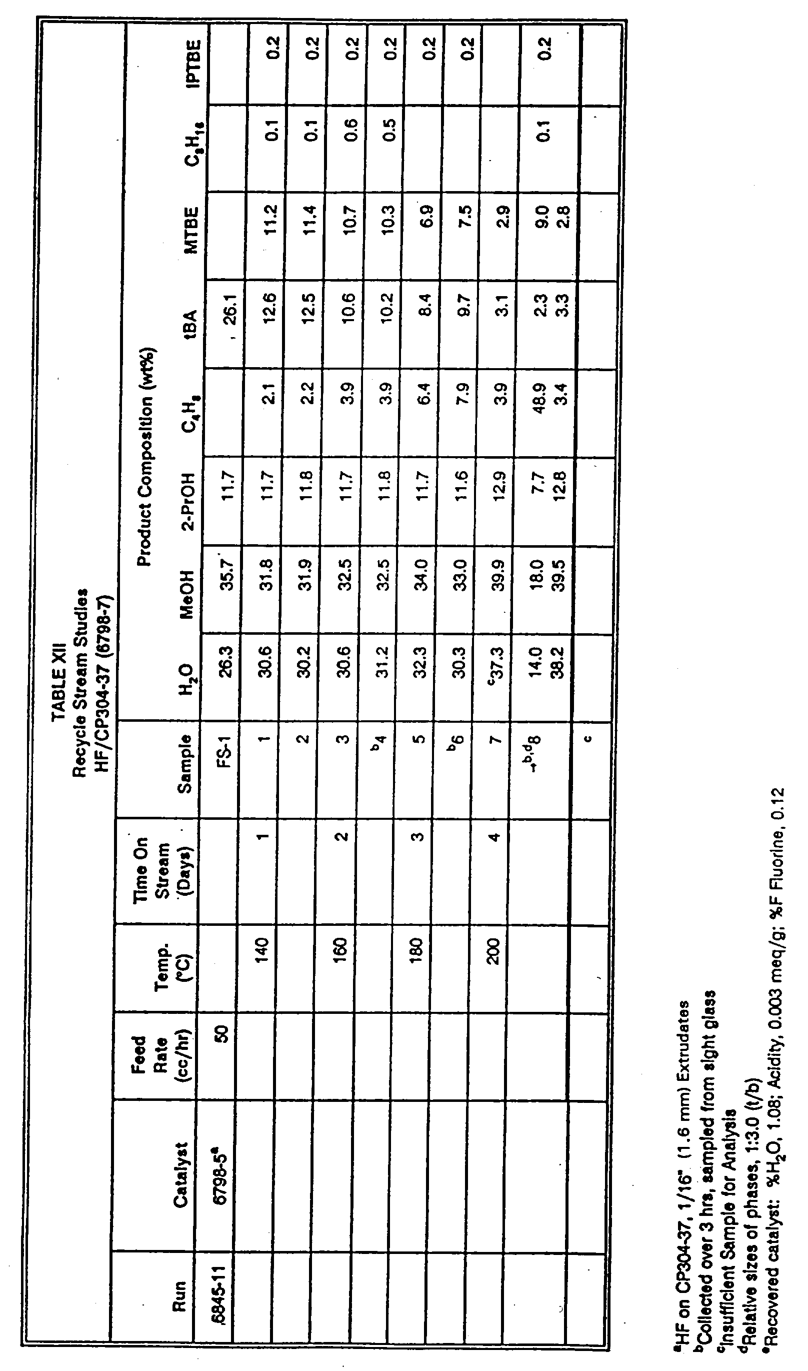

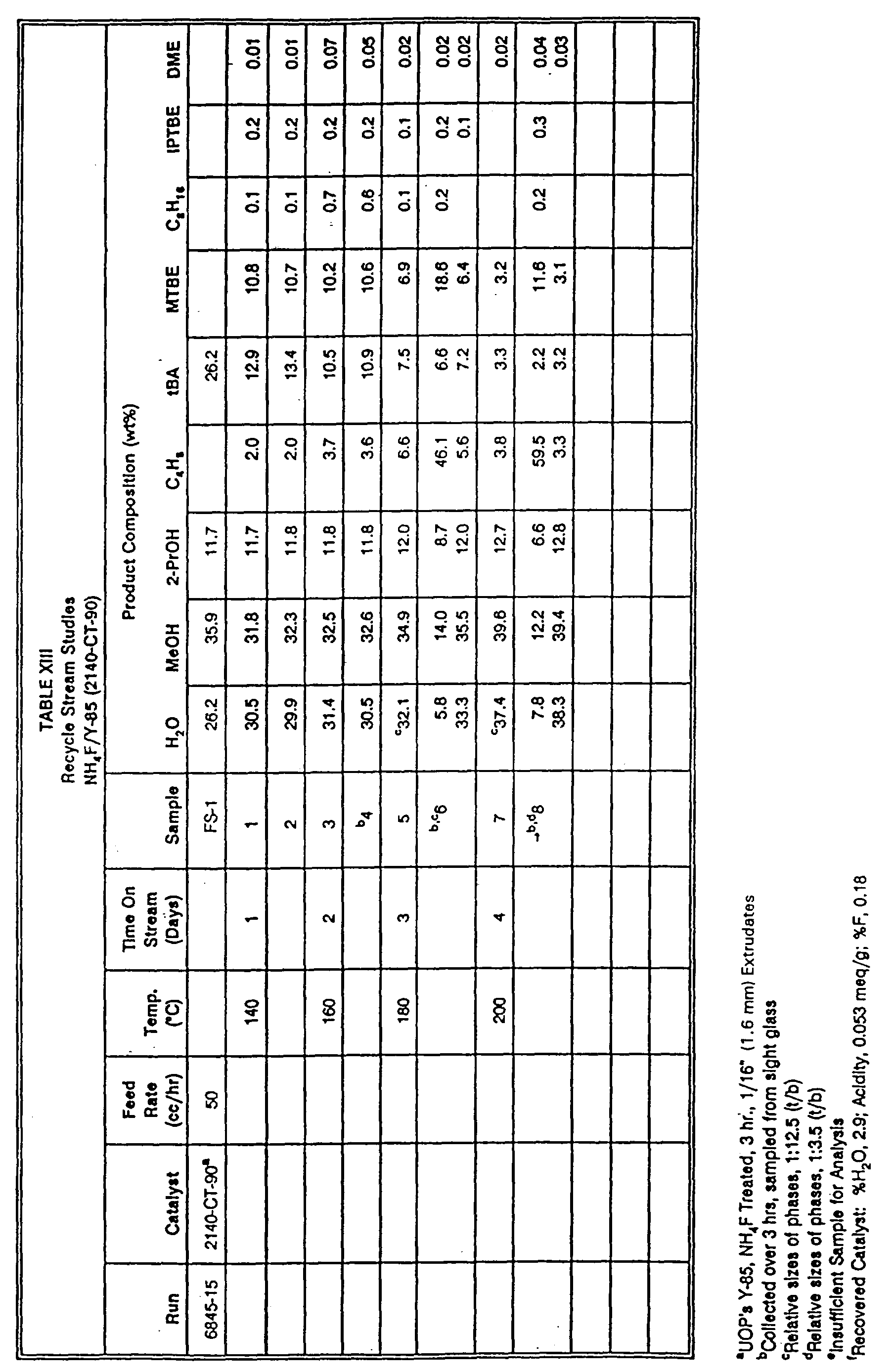

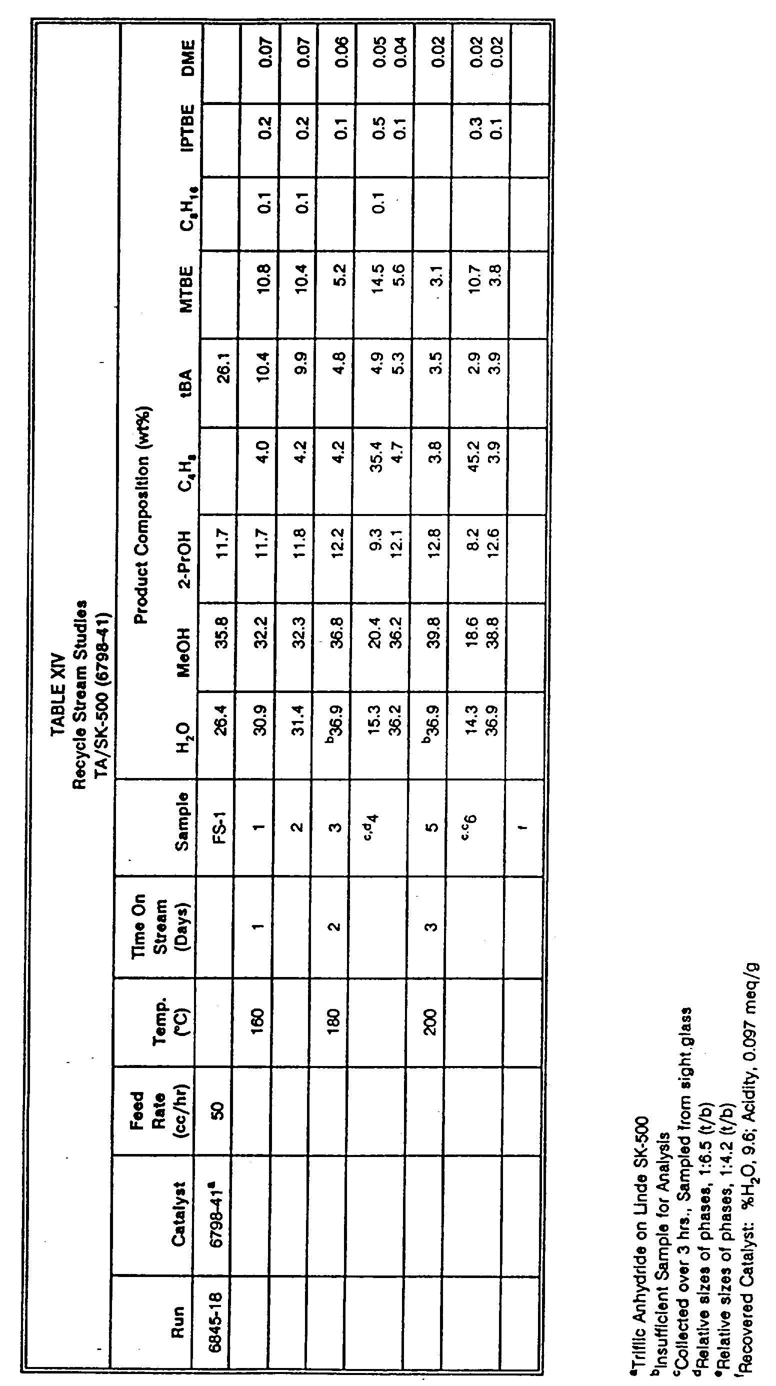

### Catalyst Life Studies

Two catalyst life studies have been made using the more active of the fluorided zeolites selected from the above screening studies. The chosen catalysts were:
a) The ammonium fluoride-treated LZY-82, Sample 2143-CT-90.
b) The HF-modified Y-zeolite CP316-26, Sample 6798-6R.

Both runs were made in the same 50cc capacity, continuous, upflow unit. Selected initial conditions were 200°C, 300 psi (2.1 MPa), LHSV 2.

Two phase product effluent compositions for run 6845-23 using the 2143-CT-90 catalyst are summarised in Table XV. Typical t-butanol conversion and MTBE/isobutylene calculated molar selectivities are given in Table III. After an initial break-in period, the DME concentrations dropped to ≤ 200 ppm, the IPTBE stabilised at ≤0.4% and the diisobutene content was often below the reliable detectable glc limit. The MTBE concentrations in the reactor effluents are plotted as a function of time on stream in Figure 1.

The second life test using HF/CP316-26 also successfully completed 2000 hours of service using the simulated second-stage feedstock. Typical two-phase product effluent analyses are given in Table XVI, the t-butanol conversion data and MTBE/isobutylene molar selectivities are given in Table IV. After an initial break-in period, the diisobutene and t-butyl isopropyl ether concentrations were typically <1.0% and ≤0.4%, respectively. Dimethyl ether effluent values were in the range 100-200 ppm. In this run the operating temperature was raised to 200°C after about 250 hours on-stream due to a loss of phase separation in sample 6945-67-2.

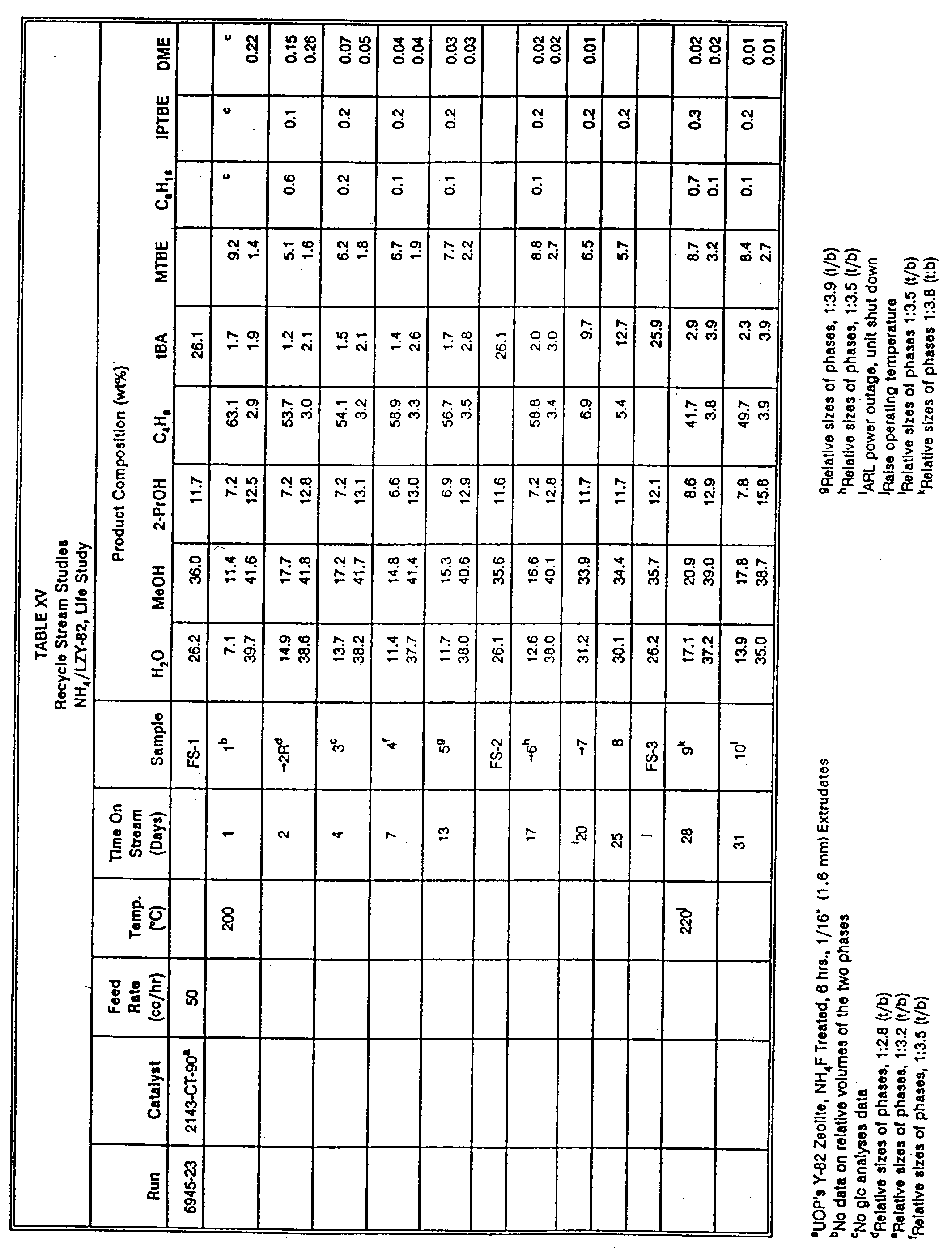

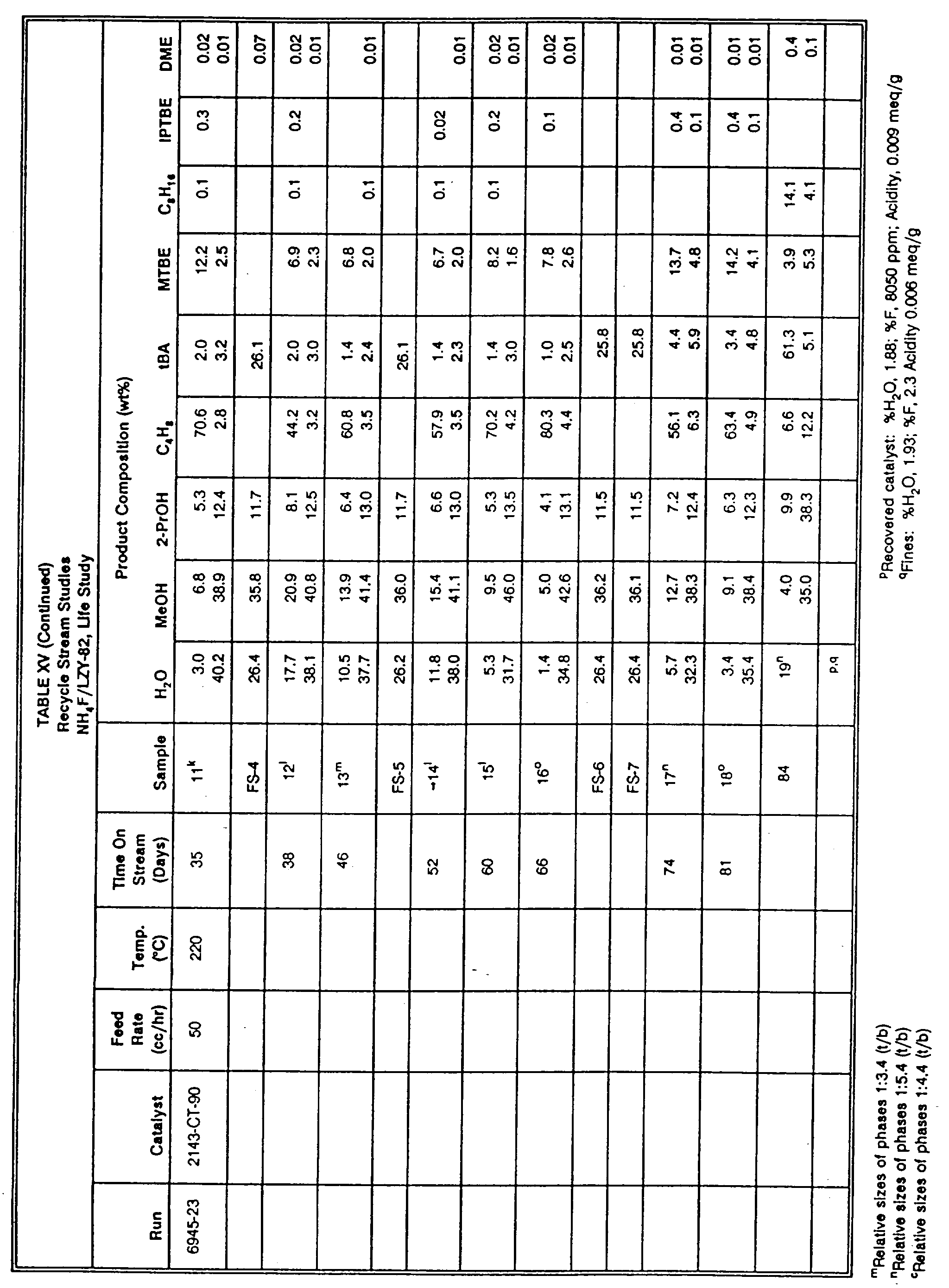

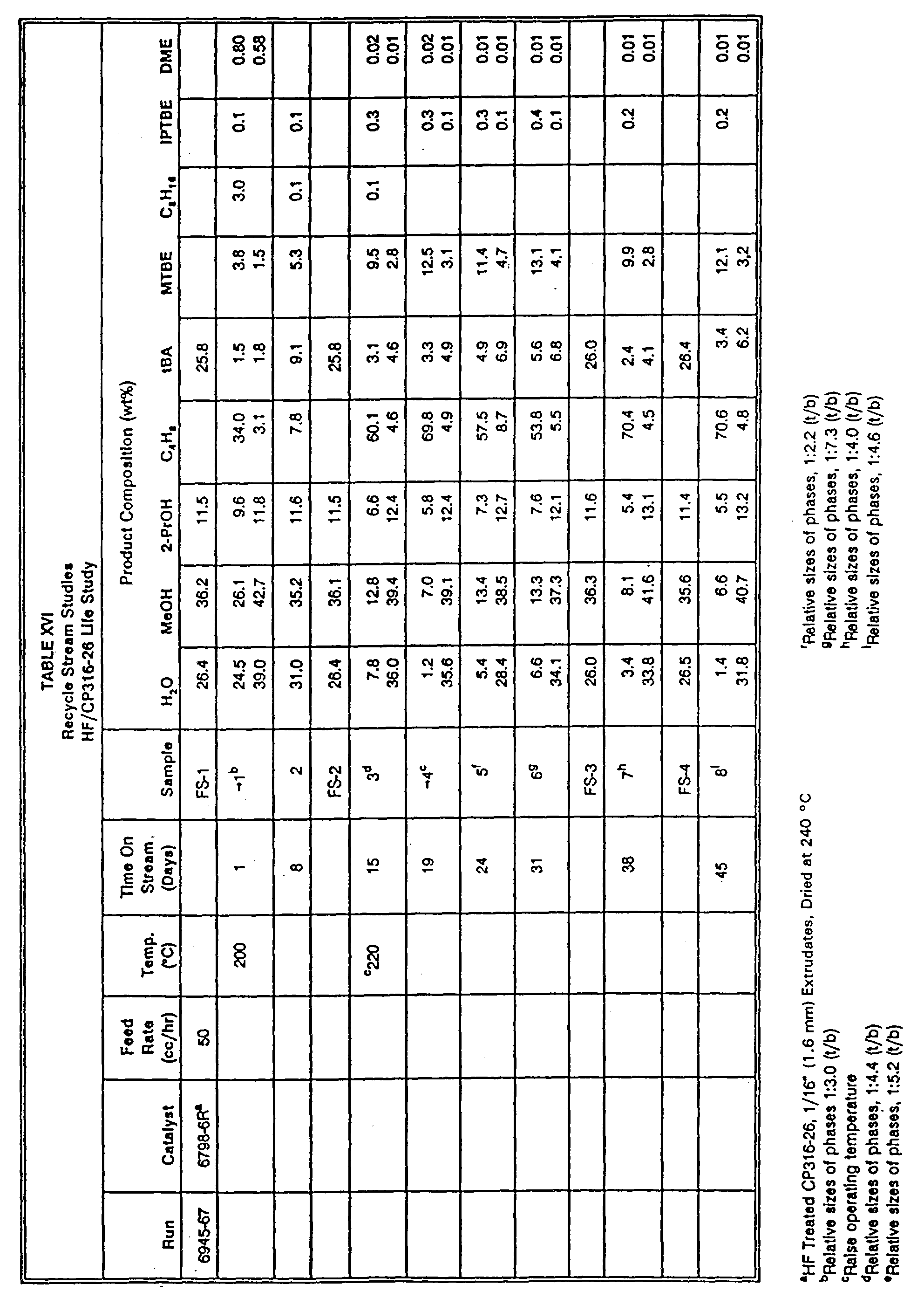

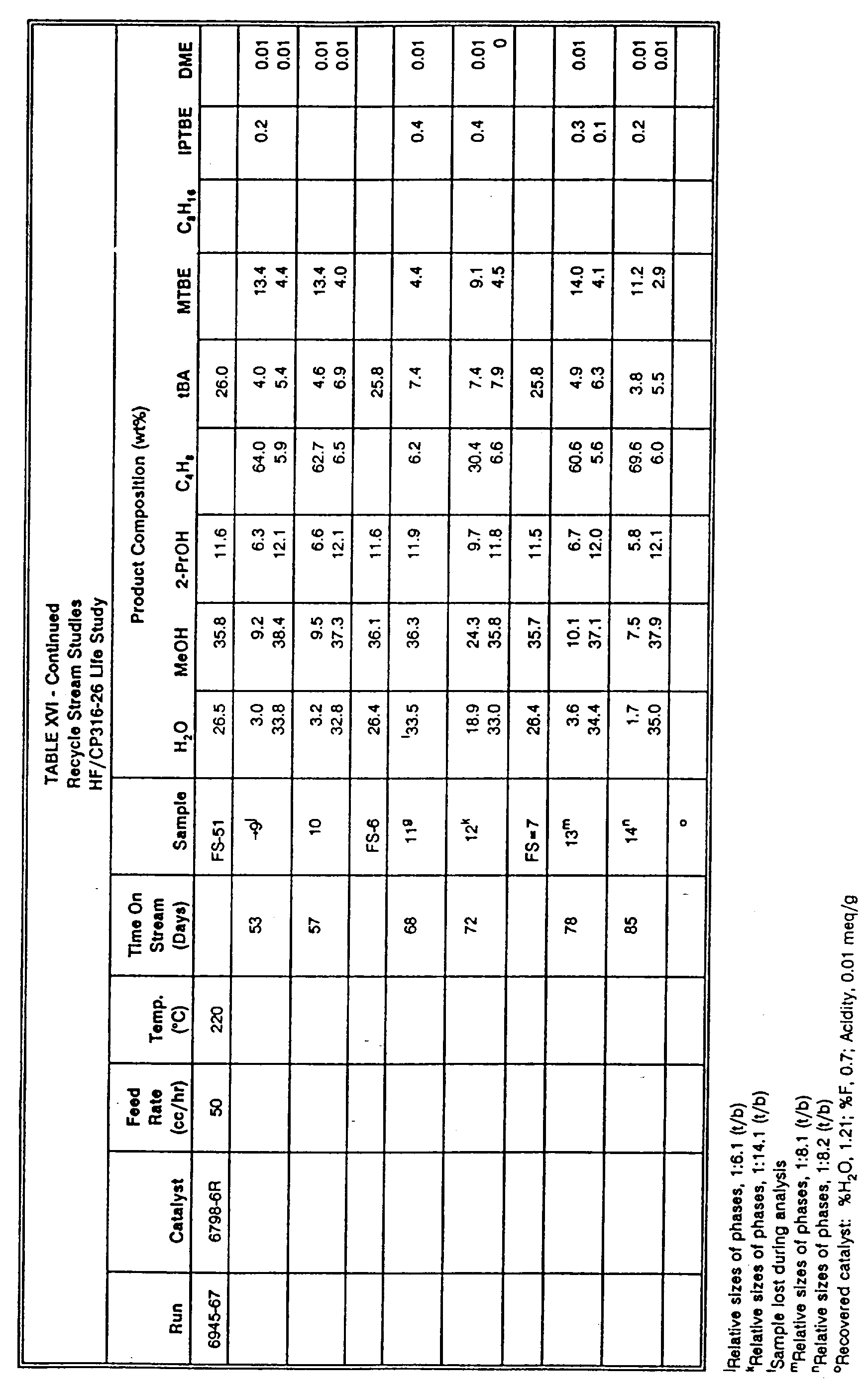

## Claims

1. A catalyst for use in the reaction between a C₁ - C₆ primary alcohol and a C₄ - C₁₀ tertiary alcohol to produce a corresponding alkyl tertiary alkyl ether, characterised in that said catalyst comprises a composition selected from:
(a) a strongly acidic Y-zeolite having a silica:alumina molar ratio of 300:1 to 1:1 and a unit cell size of 24.20 to 24.67 Å, and
(b) a silicoaluminophosphate having a pore size from 3 to 9 Å,
modified with a fluoride-containing compound and optionally mixed with a binder selected from Group III or Group IV oxides.

2. A catalyst composition as claimed in claim 1 wherein the fluorine-containing compound is selected from the group consisting of ammonium fluoride, silicon fluoride, fluorosulphonic acid and its cogeners.

3. A catalyst composition as claimed in claim 2 wherein the cogener of fluorosulphonic acid is selected from the group consisting of trifluoromethane sulphonic acid (triflic acid) and trifluoromethane sulphonic anhydride (triflic anhydride).

4. A catalyst composition as claimed in any preceding claim which contains 0.1 - 10% fluoride, 50-80% Y-zeolite, 30-50% binder.

5. A catalyst composition as claimed in any preceding claim wherein the binder is selected from alumina and zirconia.

6. A catalyst composition as claimed in claim 1 wherein the silica:alumina molar ratio is from 150:1 to 5:1.

7. A catalyst composition as claimed in any preceding claim wherein the catalyst is a fluoride-treated Y-zeolite having a silica:alumina molar ratio in the range 46:1 to 11:1 modified with ammonium fluoride.

8. A catalyst composition as claimed in any preceding claim wherein the catalyst is a modified Y-zeolite having a residual acidity in the range 0.1 to 100 mg KOH/g.

9. A catalyst composition as claimed in claim 1 which comprises a silicoaluminophosphate selected from the group consisting of SAPO-37, SAPO-5, SAPO-11 and SAPO-31.

10. A method of making a fluoride-modified etherification catalyst exhibiting improved thermal stability and extended life which comprises:
(a) treating a composition selected from
(i) a strongly acidic Y-zeolite having a silica:alumina molar ratio of 300:1 to 1:1 and a unit cell size in the range 24.20 to 24.67Å, and
(ii) a silicoaluminophosphate having a pore size from 3-9Å
with a fluoride-containing compound selected from the group consisting of hydrogen fluoride, hydrofluoric acid, ammonium fluoride, and a solution of HF or NH₄F dissolved in a suitable organic solvent;
(b) heating to 100-250°C for 2 to 24 hours, and
(c) calcining at 400 to 700°C for 0.5 to 5 hours.
